# NEUE EUROPÄISCHE PATENTSCHRIFT

(11) **EP 2 277 597 B2**
(45) Veröffentlichungstag und Bekanntmachung des Hinweises auf die Entscheidung über den Einspruch: **26.06.2024**
(45) Hinweis auf die Patenterteilung: 16.01.2019
(21) Anmeldenummer: 10177126.9
(22) Anmeldetag: 19.03.2007
(51) Int. Cl.: A61Q 5/08, A61K 8/41, A61K 8/46, A61K 8/49, D21H 21/32, D06P 1/62, D06P 5/13

(54) **Reduktiver Farbabzug**
Reductive colour removal
Décoloration réductive

(30) Priorität: 11.05.2006 DE 102006022274; 21.03.2006 DE 102006013260
(43) Veröffentlichungstag der Anmeldung: 26.01.2011
(62) Teilanmeldung aus: 07723368.2
(73) Patentinhaber: Henkel AG & Co. KGaA, 40589 Düsseldorf (DE)
(72) Erfinder: Müller, Burkhard, 21075, Hamburg (DE); Neubueser, Inge, 22587, Hamburg (DE); Groß, Wibke, 41836, Hückelhoven (DE)

(56) Entgegenhaltungen:
- EP-A2- 2 001 559
- FR-A1- 2 814 948
- FR-A1- 2 829 764
- US-A- 3 892 845
- US-B1- 6 171 347

## Beschreibung

Die Erfindung betrifft Zusammensetzungen zum reduktiven Farbabzug, welche neben organischen Sulfinsäurederivaten mindestens eine Verbindung mit mindestens einem quartären Stickstoffatom, enthalten. Diese Farbabzugsmittel eignen sich für die Entfärbung keratinhaltigen Fasern, beispielsweise menschlicher Haare. Gleichfalls ist ein Verfahren zur Entfärbung keratinhaltigen Fasern, sowie die Verwendung besagter Mittel zum Farbabzug Gegenstand der Erfindung.

Beim Färben wird der Farbstoff auf das Substrat durch Adsorption an die Oberfläche, durch Eindiffundieren, durch Bildung auf und/oder in dem Substrat bzw. durch chemische Bindung übertragen. Für das Färben, beispielsweise von Papier, Textilien oder keratinhaltigen Fasern, kommen im allgemeinen entweder direktziehende Farbstoffe oder Oxidationsfarbstoffe in Frage. Oxidationsfarbstoffe entstehen durch oxidative Kupplung einer oder mehrerer Entwicklerkomponenten untereinander oder mit einer oder mehreren Kupplerkomponenten. Kuppler- und Entwicklerkomponenten werden auch als Oxidationsfarbstoffvorprodukte bezeichnet. Die oxidative Kupplung findet bevorzugt während der Färbevorgangs statt, damit die Farbstoffvorprodukte in das Substrat hinein diffundieren können und der Farbstoff sich in dem Substrat bildet. Durch die Größe des entstandenen Farbstoffmoleküls wird ein Auswaschen aus dem Substrat erschwert.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.
Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxy-pyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Unter direktziehenden Farbstoffen werden im allgemeinen Farbstoffe verstanden, die bereits vor Beginn des Färbens vorgebildet sind und auf das Substrat aufziehen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Ein wichtiges technisches Gebiet stellt neben dem Färben das Abziehen von Farbstoffen dar. Darunter versteht man im Allgemeinen das Entfernen von Färbungen oder Bedruckungen durch Auswaschen, chemische Veränderung oder Zerstörung des Farbstoffes. Die oxidative oder die reduktive Entfärbung gefärbter Materialien findet insbesondere bei der Entfärbung von Textilien oder Haaren Anwendung.
Eine oxidative Entfärbung führt meist zu guten Ergebnissen. Jedoch kann durch die starke Oxidationswirkung des zur Entfärbung benutzten Oxidationsmittels die Struktur des Substrats chemisch verändert werden. Dies geht mit einer unerwünschten physikalischen Änderung des Substrats einher. Textilien oder Haare können beispielsweise spröde werden oder insbesondere bei mehrmaliger Entfärbung gar brechen. Der visuelle Eindruck, die Haptik aber auch die Haltbarkeit des Substrats werden dadurch negativ beeinflusst.
Weniger Einfluss auf die Substratstruktur, insbesondere auf die Struktur keratinhaltiger Fasern, nehmen reduktive Entfärbemittel. Die reduktiven Entfärbemittel entfärben kaum die Naturhaarfarbe, sondern wirken lediglich auf die durch Färbung aufgetragenen Farbstoffe reduktiv ein. Es tritt somit kaum eine Aufhellung des Haars auf. Allerdings bedürfen die reduktiven Entfärbemittel einer Verbesserung der Entfärbeleistung.
Überwiegend nutzt man reduktiv wirkende Schwefelverbindungen zur Entfärbung. Bekanntermaßen eignen sich Dithionite oder Derivate der 1-Hydroxymethylsulfinsäure bzw. der 1-Aminomethylsulfinsäure als Reduktionsmittel.

Aus der Druckschrift US-A-3 892 845 sind dem Fachmann reduktive Farbabzugsmittel bekannt, mit deren Hilfe sich gefärbte keratinhaltige Fasern entfärben lassen. Als Reduktionsmittel ist 1-Hydroxymethylsulfinsäure oder ein Salz davon in den Entfärbemitteln enthalten. Gemäß DE-OS-1 617 829 lässt sich durch Zugabe von 1-Hydroxymethylsulfinsäure zu oxidativen Entfärbemitteln, enthaltend Wasserstoffperoxid und Persulfate, deren Bleichwirkung verstärken. Laut Offenbarung der EP-A-1 079 018 eignen sich Aminoalkansulfinate der Formel R¹_{3-z}N (CR²R³-SO₂-M)_{z} (R², R³ = Wasserstoff oder (C₁ bis C₄)-Alkyl) zur teilweisen Entfärbung von mit Küpenfarbstoffen oder Schwefelfarbstoffen gefärbten oder bedruckten Textilien. Gemäß WO-A1-99/18067 eignen sich 1-Hydroxyalkylsulfinsäuren bzw. 1-Aminoalkylsulfinsäuren, welche eine Carboxygruppe, eine Sulfonsäuregruppe, eine Acylgruppe, eine Aminocarbonylgruppe oder eine Alkoxycarbonylgruppe tragen, zur Entfärbung gefärbter Substrate. Besagte Derivate besitzen eine vergleichbare oder bessere Entfärbekraft wie die 1-Hydroxymethylsulfinsäure (*vide supra*). In der WO-A1-02/30369 wird die Entdeckung beschrieben, dass sich mit den SulfinsäureDerivaten der WO-A1-99/18067 auch keratinhaltige Fasern, wie beispielsweise Haare, entfärben lassen. Weitere zur Entfärbung keratinhaltiger Fasern geeignete Sulfinsäurederivate finden sich in den Druckschriften WO-A1-03/026597 und WO-A1-03/41668. In der US 3 892 845 A wird der reduktive Farbabzug unter Verwendung einer Kombination von Hydroxymethansulfinaten oder Hydrosulfiten und Betainen offenbart. Weiterhin offenbart die FR 2 829 764 A1 die Verwendung von Sulfinsäurederivaten mit mindestens zwei Carboxyalkylgruppen zur Entfärbung von keratinischen Fasern. Schließlich wird in der FR 2 814 948 A1 die Entfärbung von keratinischen Fasern unter Verwendung bestimmter Sulfinsäurederivate offenbart. Den drei zuletzt genannten Dokumenten lässt sich jedoch kein Einfluss des Einsatzes bestimmter kationischer Tenside auf die Verminderung der Nachdunklung sowie die Verbesserung der toxikologischen Eigenschaften entnehmen. Substrate, die mit den Sulfinsäurederivaten des Standes der Technik reduktiv entfärbt wurden, erfahren einige Zeit nach dem Farbabzug eine unerwünschte Nachdunkelung.

Ein erster Gegenstand der Erfindung sind daher Mittel, enthaltend in einem Träger mindestens ein Sulfinsäurederivat der Formel (I) 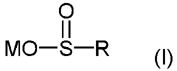 worin
- M: steht für ein Wasserstoffatom oder ein Äquivalent eines ein- oder mehrwertigen Kations,
- R: für einen Rest gemäß der Formel (II) steht,

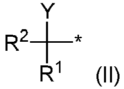

worin bedeuten
   - Y: unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
   - R¹: unabhängig voneinander ein Wasserstoffatom, eine (C₁ bis C₆)-Alkylgruppe oder eine Carboxyalkylgruppe -(CH₂)ₘ-COOM", in der M" für ein Wasserstoffatom oder für ein Äquivalent eines ein- oder mehrwertigen Kations steht und m die Zahl 0, 1 oder 2 bedeutet,
   - R²: ein Wasserstoffatom, eine (C₁ bis C₆)-Alkylgruppe, einen Carboxyalkylrest -(CH₂)ₙ-COOM^{III} mit
   M^{III} = Wasserstoffatom oder ein Äquivalent eines ein- oder mehrwertigen Kations und n eine ganze Zahl 0, 1, 2, 3, 4, 5 oder 6,
   einen (C₁ bis C₆)-Alkyloxycarbonylrest, eine N,N,N-Tri[(C₁ bis C₆)-alkyl]ammonium-(C₁ bis C₆)-alkylgruppe eine Carboxy-(C₂ bis C₆)-alkenylgruppe, eine Cyano-(C₁ bis C₆)-alkylgruppe oder eine (C₁ bis C₆)-Alkoxycarbonyl-(C₁ bis C₆)-alkylgruppe, oder
   R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird, oder
   einen aliphatischen oder aromatischen Heterozyklus, der substituiert sein kann,
mit der Maßgabe, dass in Formel (II) R¹ und R² nicht gleichzeitig für ein Wasserstoffatom stehen,
in Kombination mit mindestens einer Verbindung, besitzend mindestens ein quartäres Stickstoffatom, wobei die Verbindungen, besitzend mindestens ein quartäres Stickstoffatom ausgewählt werden aus Verbindungen mit der Formel (Q1-c),

(R¹⁰)_{y}(Me)(_{4-y})N⁺ X⁻ (Q1-c)

worin
- R¹⁰ unabhängig voneinander eine (C₈- bis C₃₀)-Alkylgruppe bedeutet,
- y gleich 1 oder 2 ist und
- X⁻ ein physiologisch verträgliches organisches oder anorganisches Anion ist.

Aufgabe der vorliegenden Erfindung ist es, reduktive Entfärbemittel bereitzustellen, die das Substrat dauerhaft ohne Nachdunkelung entfärben. Die Substratstruktur soll dabei geschont werden. Ferner sollten die in den Entfärbemitteln eingesetzten Reduktionsmittel für eine kosmetische Verwendung physiologisch verträglich und toxikologisch unbedenklich sein.

Überraschenderweise wird die Aufgabe durch Mittel gelöst, die eine Kombination mindestens eines der erfindungsgemäßen organischen Sulfinsäurederivate mit mindestens einer erfindungsgemäßen Verbindung enthaltend mindestens ein quartäres Stickstoffatom enthalten. Die Entfärbung gefärbter keratinhaltiger Fasern, insbesondere menschliches Haar, gelingt sehr gut ohne dass sich nach dem Entfärbevorgang eine Nachdunkelung in dem hohen Maße einstellt, wie sie durch die Verwendung der Sulfinsäurederivate allein hervorgerufen wird. Die erfindungsgemäßen Mittel eignen sich besonders zur faserschonenden Entfärbung keratinhaltiger Fasern.

Unter keratinhaltigen Fasern sind beispielsweise Wolle, Pelze, Federn und insbesondere menschliche Haare zu verstehen.

Falls die Verbindungen gemäß Formel (I) mindestens ein Chiralitätszentum enthalten, sind selbstverständlich alle Stereoisomere allein sowie deren Mischungen, insbesondere deren Racemate, erfindungsgemäß.

Die Verbindungen gemäß Formel (I) können neben der Form als freie Säure oder als dessen Salz auch als inneres Salz vorliegen, insbesondere dann, wenn neben der Sulfinat-Gruppe der Formel (I) zusätzlich ein kationischer Substituent (siehe Definition R²) im Molekül enthalten ist.

Wenn die Verbindung der Formel (I) als Säure vorliegt, bedeuten die Reste M, M" und/oder M^{III} ein Wasserstoffatom. Die Fragmente MO- der Formel (I) und M"O- bzw. M^{III}O- gemäß R¹ bzw. R² aus Formel (II) bilden in diesem Fall eine Hydroxygruppe. Wenn die Sulfinsäure der Formel (I) als Salz vorliegt, steht mindestens einer der Reste M, M" oder M^{III} für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung 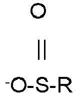 vorliegenden Sulfinat-Fragments aus Formel (I) bzw. der Carboxylat-Fragmente der Reste aus R¹ bzw. R² der Formel (II). Das dafür zu verwendende Äquivalent des entsprechenden Kations beträgt 1/z. Das Fragment MO- der Formel (I) steht im Fall der Salzbildung für die Gruppe: 1/z (M^{z+}) -O-. Gleiches gilt mutatis mutandis für M^{II} und M^{III}.

Als entsprechende ein- oder mehrwertige Kationen kommen prinzipiell alle Kationen in Frage, die keine Redox-Reaktion mit dem übrigen Sulfinat-Fragment der Formel (I) eingehen. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (I) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M, M^{II} und M^{III} stehen bevorzugt unabhängig voneinander für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Das Äquivalent des gegebenenfalls vorhandenen ein- oder mehrwertigen Anions gemäß Formel (I) wird, analog zur Definition der Kationäquivalente, zur Wahrung der Elektroneutralität durch Formulierung eines stöchiometrischen Koeffizienten kleiner 1 vor der Bezeichnung des Anions beschrieben. Die besagten Anionen werden im Weiteren definitionsgemäß mit An- symbolisiert. Sie werden bevorzugt ausgewählt aus Halogenid, ½ Sulfat, Hydrogensulfat, ½ Carbonat, Hydrogencarbonat, 1/3 Phosphat, ½ Hydrogenphosphat, Dihydrogenphosphat oder p-Toluolsulfonat. Besonders bevorzugt steht das Anion für Chlorid, Bromid, p-Toluolsulfonat oder Hydrogensulfat.

Erfindungsgemäß sind weiterhin solche Sulfinsäurederivate gemäß Formel (I) bevorzugt, in denen der Rest Y der Formel (II) eine Hydroxygruppe oder eine Gruppe -NH₂ bedeuten.

Sulfinsäurederivate, in denen R¹ für ein Wasserstoffatom oder eine Methylgruppe steht, sind erfindungsgemäß bevorzugt.

Der Rest R der Formel (I) steht für einen Rest der oben genannten Formel (II), daher ergibt sich aus der Formel (I) das erfindungsgemäße Sulfinsäurederivat der Formel (Ia), 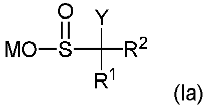 worin M, Y, R¹ und R² die unter den Formeln (I) und (II) definierte Bedeutung haben. Die zuvor erwähnten bevorzugten Definitionen der Reste M, Y und R¹ gelten auch hier, allein oder gemeinsam auf Formel (Ia) angewendet, als bevorzugt.

Es ist erfindungsgemäß, wenn gemäß einer bevorzugten Ausführungsform der Erfindung der Rest R² gemäß Formel (II) bzw. Formel (Ia) für einen aliphatischen oder aromatischen Heterozyklus steht, welcher gegebenenfalls substituiert sein kann. Falls die oben genannten ausgewählten aliphatischen oder aromatischen Heterozyklen dieser Ausführungsform substituiert sind, dann sind diese bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido, substituiert.

Im Rahmen dieser Ausführungsform ist es wiederum bevorzugt, die aliphatischen oder aromatischen Heterozyklen des Rests R² auszuwählen aus Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl oder Chinazolinyl, welche gegebenenfalls substituiert sein können, bevorzugt mit den oben genannten Substituenten, besonders bevorzugt mit mindestens einem Rest aus (C₁ bis C₆)-Alkyl, (C₁ bis C₆)-Hydroxyalkyl, Hydroxy, Amino, (C₁ bis C₆)-Alkoxy, Carboxy, Halogenatom, Nitrogruppe oder Sulfonsäurerest.

Dabei sind besonders bevorzugte Sulfinsäurederivate dieser Ausführungsform Verbindungen der nachfolgenden Formeln (Ia-1) bis (Ia-4) 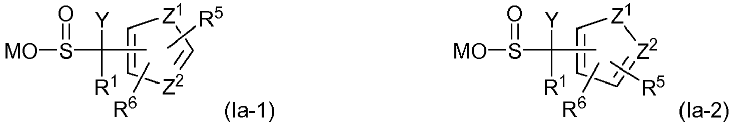 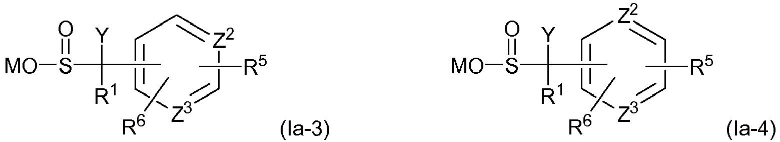 worin
- M, Y und R¹: die unter Formel (I) definierten Bedeutungen haben,
- Z¹: fürein Sauerstoffatom, ein Schwefelatom oder eine Gruppe N-R^{IV} steht, worin R^{IV} ein Wasserstoffatom, eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe bedeutet,
- Z² und Z³: unabhängig voneinander für eine Gruppe CH oder für ein Stickstoffatom stehen,
- R⁵ und R⁶: unabhängig voneinander stehen für ein Wasserstoffatom, eine (C₁ bis C₆)-Alkylgruppe, eine (C₂ bis C₆)-Alkenylgruppe, eine (C₁ bis C₆)-Hydroxyalkylgruppe, eine Hydroxygruppe, eine (C₁ bis C₆)-Alkoxygruppe, eine Aminogruppe, eine Di(C₁ bis C₆)alkylaminogruppe, eine Nitrogruppe, eine Halogenatom, eine Carbamoylgruppe, eine Sulfonamidogruppe, eine Cyanogruppe oder eine Carboxamidogruppe oder gemeinsam eine Benzanellierung bilden, welche wiederum substituiert sein kann.

Die Gruppe N-R^{IV} aus Rest Z¹ bildet im Ring des Heterozyklus eine Azandiylgruppe -NR^{IV}-. Die Gruppe CH der Reste Z² bzw. Z³ bildet im Ring des Heterozyklus eine Methanylylidengruppe =CH-, die selbstverständlich auch mit einem der Reste R⁵ bzw. R⁶ substituiert sein kann.

Wenn in den Formeln (Ia-1) bzw. (Ia-2) Z¹ für ein Sauerstoffatom, Z² für eine Gruppe CH und Y für eine Hydroxygruppe steht, so ist es erfindungsgemäß bevorzugt, wenn in den Formeln (Ia-1) bzw. (Ia-2) R⁵ und R⁶ nicht gleichzeitig für ein Wasserstoffatom stehen.

Die zuvor erwähnten bevorzugten Definitionen der Reste M, Y und R¹ gelten auch hier, allein oder gemeinsam, auf Formeln (Ia-1) bis (Ia-4) angewendet, als bevorzugt.

Die Benzanellierung aus den Resten R⁵ und R⁶ der Formel (Ia-1) bis (Ia-4) ist, wenn sie substituiert ist, bevorzugt mit mindestens einem Rest aus (C₁ bis C₆)-Alkyl, (C₂ bis C₆)-Alkenyl, (C₁ bis C₆)-Hydroxyalkyl, Aryl-(C₁ bis C₆)-alkyl, Hydroxy, (C₁ bis C₆)-Alkoxy, Amino, Di(C₁ bis C₆)alkylamino, Nitro, Halogen, Carbamoyl, Sulfonamido, Cyano oder Carboxamido substituiert. R⁵ und R⁶ gemäß Formeln (Ia-1) bis (Ia-4) stehen besonders bevorzugt für ein Wasserstoffatom, eine Hydroxygruppe, eine (C₁ bis C₆)-Alkylgruppe, eine (C₁ bis C₆)-Alkoxygruppe, eine (C₁ bis C₆)-Hydroxyalkylgruppe, Halogenatom oder Nitrogruppe.

Wenn die Reste R¹ und R² gemäß Formel (II) im Rahmen der ersten Ausführungsform gemeinsam mit dem Restmolekül einen aliphatischen 5-, 6-, oder 7-gliederigen Ring bilden, welcher mindestens ein kationisches, quaterniertes Stickstoffatom als Heteroatom enthält wobei die kationische Ladung gegebenenfalls durch die ein Äquivalent eines ein oder mehrwertigen Anions kompensiert wird, sind folgende Verbindungen gemäß Formeln (Ia-5) und (Ia-6) bevorzugt, 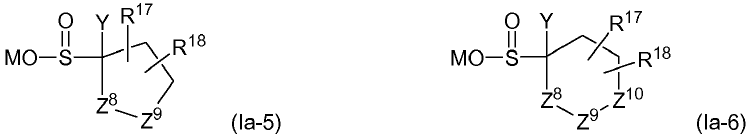 worin
- Y: die unter Formel (I) beschriebenen Definitionen bedeutet,
- M: die unter Formel (I) beschriebenen Definitionen oder eine negative Ladung bedeutet,
- Z⁸, Z⁹ und Z¹⁰: einer dieser Reste eine Azoniumdiyl-Gruppe N⁺R^{V}R^{VI} bedeutet mit R^{V} und R^{VI} stehen unabhängig voneinander für eine (C₁ bis C₆)-Alkylgruppe, eine (C₂ bis C₆)-Alkenylgruppe, eine (C₁ bis C₆)-Hydroxyalkylgruppe oder eine Aryl-(C₁ bis C₆)-alkylgruppe, und die übrigen dieser Reste für eine CH₂-Gruppe stehen,
- R¹⁷ und R¹⁸: unabhängig voneinander ein Wasserstoffatom, eine (C₁ bis C₆)-Alkylgruppe, eine Hydroxygruppe, ein Halogenatom oder eine Carboxygruppe bedeuten,
mit der Maßgabe, dass ein Äquivalent eines ein- oder mehrwertigen Anions zugegen ist, wenn M keine negative Ladung bedeutet.

Die CH₂-Gruppe bildet im Ring des Heterozyklus ein Methandiylfragment -CH₂-, das selbstverständlich auch mit einem der Reste R¹⁷ bzw. R¹⁸ substituiert sein kann.

Die zuvor erwähnten bevorzugten Definitionen der Reste M und Y gelten auch hier, allein oder gemeinsam, auf Formeln (Ia-5) und (Ia-6) angewendet, als bevorzugt. Die unter der Maßgabe beschriebene Bedingung beschreibt die Ausbildung eines inneren Salzes, das ebenso eine bevorzugte Ausführungsform darstellt.

Weiterhin erfindungsgemäß bevorzugt eingesetzte Verbindungen der Formel (I) sind Verbindungen der Formel (Ia-8) 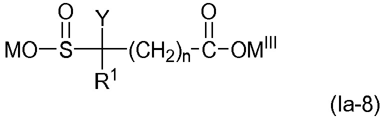 worin M, Y, R¹, n und M^{III} die unter Formel (I) und (II) genannten Bedeutungen haben. Die zuvor erwähnten bevorzugten Definitionen der Reste M, Y, R¹, n, und M^{III} gelten auch hier, allein oder gemeinsam auf Formel (Ia-8) angewendet, als bevorzugt.

Es sind darüber hinaus solche Verbindungen der Formel (Ia-8) erfindungsgemäß bevorzugt, bei welchen Y eine Hydroxy- oder Aminogruppe bedeutet.

Bevorzugt steht n in Formel (Ia-8) für eine Zahl 0, 1 oder 2.

Im Folgenden sollen Beispiele für die als Substituenten im Rahmen dieser Anmeldung genannten Gruppen bzw. Reste erwähnt werden. Beispiele für (C₁ bis C₆)-Alkylreste sind lineare, verzweigte oder zyklische (C₁ bis C₆)-Alkylgruppen, wobei lineare oder verzweigte (C₁ bis C₆)-Alkylgruppen bevorzugt sind. Insbesondere sind die Gruppen Methyl, Ethyl, Propyl, Isopropyl, n-Butyl, sec-Butyl, tert.-Butyl, n-Pentyl und n-Hexyl geeignet. Beispiele für entsprechend geeignete zyklische Alkylgruppen sind Cyclopentyl und Cyclohexyl.

Beispiele für bevorzugte (C₂ bis C₆)-Alkenylreste sind Vinyl, Allyl und Butenyl.

Erfindungsgemäß bevorzugte (C₁ bis C₆)-Alkoxyreste sind beispielsweise eine Methoxy- oder eine Ethoxygruppe.

Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für (C₁ bis C₆)-Alkoxycarbonylgruppen; die Methoxycarbonyl- und die Ethoxycarbonylgruppe sind dabei besonders bevorzugt.

Die 2-Methoxycarbonylethyl-, 2-Ethoxycarbonylethyl-, 2-Propoxycarbonylethyl-, 2-Isopropoxycarbonylethyl-, 2-Butoxycarbonylethyl-, 2-sec-Butoxycarbonylethyl-, 2-tert-Butoxycarbonylethyl-, 3-Methoxycarbonylpropyl-, 3-Ethoxycarbonylpropyl-, 3-Propoxycarbonylpropyl-, 3-Isopropoxycarbonylpropyl-, 3-Butoxycarbonylpropyl-, 3-sec-Butoxycarbonylpropyl- und 3-tert-Butoxycarbonylpropylgruppe sind Beispiele für (C₁ bis C₆)-Alkoxycarbonyl-(C₁ bis C₆)-alkylgruppen.

Beispiele für erfindungsgemäße Cyano-(C₁ bis C₆)-alkylgruppen sind Cyanomethyl, 2-Cyanoethyl, 3-Cyanopropyl, 4-Cyanobutyl und 5-Cyanopentyl.

Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe genannt werden. Eine 2-Hydroxyethylgruppe ist besonders bevorzugt.

Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe.

Die Methoxyethyl-, Ethoxyethyl-, Methoxypropyl-, Methoxybutyl-, Ethoxybutyl- und die Methoxyhexylgruppe sind Beispiele für erfindungsgemäße (C₁ bis C₆)-Alkoxy-(C₁ bis C₆)-alkylgruppen.

Eine bevorzugte Hydroxy-(C₁ bis C₆)-alkoxygruppe ist die 2-Hydroxyethoxygruppe. Bevorzugte Arylgruppen sind Phenyl, Naphthyl und Biphenyl.

Bevorzugte Heteroarylgruppen sind Thienyl, Furyl, Pyrrolyl, Imidazolyl, Thiazolyl, Oxazolyl, Pyrazolyl, Pyridyl, Pyrimidinyl, Pyrazyl, Pyridazyl, Benzimidazolyl, Benzothiazolyl, Benzoxazolyl, Indolyl, Chinolinyl, Chinoxalinyl und Chinazolinyl. Die Trifluomethyl- und die Pentafluorethylgruppe sind bevorzugte Perfluor-(C₁ bis C₆)-alkylgruppen.

Beispiele für Halogenatome sind F-, Cl-, Br- oder I-Atome, wobei Cl- und Br-Atome ganz besonders bevorzugt sind. Bevorzugte Aryl-(C₁ bis C₆)-alkylgruppen sind Benzyl und 2-Phenylethyl.

Die Trimethylammonium- und Diethylmethylammonium- sind Beispiele für eine Gruppe - N⁺R^{I}R^{II}R^{III}.

Die 2-Trimethylammoniumethyl- ist ein Beispiel für eine N,N,N-Tri[(C₁ bis C₆)-akyl]ammonium-(C₁ bis C₆)-alkylgruppe. Eine bevorzugte (C₁ bis C₆)-Carboxyalkylgruppe ist die 3-Carboxypropylgruppe.

Die weiteren verwendeten Begriffe leiten sich erfindungsgemäß von den hier gegebenen Definitionen ab.

Ganz besonders bevorzugte Vertreter der Sulfinsäurederivate gemäß Formel (I) sind die Sulfinsäuren gemäß folgender Liste oder deren Salze mit einem Äquivalent mindestens eines ein- oder mehrwertigen Kations:

| Name: | korrespondierende Struktur: |
|---|---|
| 2-Furyl(amino)methansulfinsäure, | 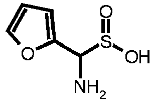 |
| Amino(thien-2-yl)methansulfinsäure, | 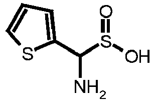 |
| Amino(1*H*-imidazol-2-yl)methansulfinsäure, | 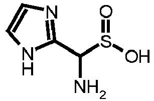 |
| Amino(1,3-thiazol-2-yl)methansulfinsäure, | 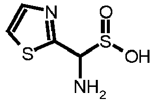 |
| Amino(1,3-oxazol-2-yl)methansulfinsäure, | 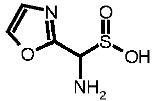 |
| Amino(1*H*-pyrrol-2-yl)methansulfinsäure, | 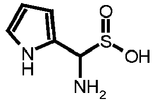 |
| Amino(hydroxyl-2-yl)methansulfinsäure, | 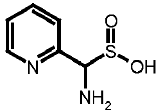 |
| Amino(hydroxyl-4-yl)methansulfinsäure, | 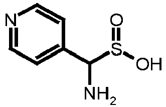 |
| Amino[3-hydroxy-5-(hydroxymethyl)-2-methylpyridin-4-yl]methansulfinsäure, | 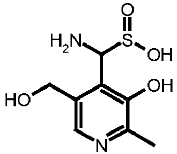 |
| Amino(chinolin-2-yl)methansulfinsäure, | 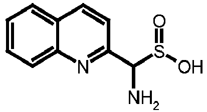 |
| Amino(chinolin-4-yl)methansulfinsäure, | 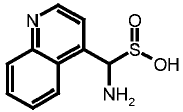 |
| 1*H*-Benzimidazol-2-yl(amino)methansulfinsäure, | 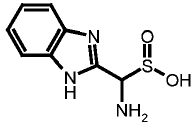 |
| 1,3-Benzothiazol-2-yl(amino)methansulfinsäure, | 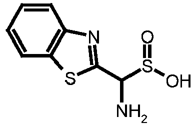 |
| 1,3-Benzoxazol-2-yl(amino)methansulfinsäure, | 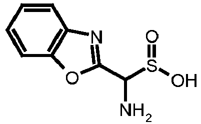 |
| Hydroxy(thien-2-yl)methansulfinsäure, | 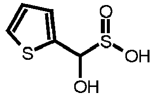 |
| Hydroxy(thien-3-yl)methansulfinsäure | 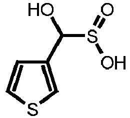 |
| (3-Bromthien-2-yl)(hydroxyl)methansulfinsäure, | 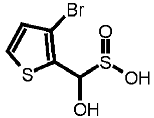 |
| Hydroxy(1H-imidazol-2-yl)methansulfinsäure, | 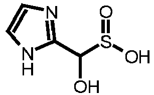 |
| Hydroxy(1H-imidazol-5-yl)methansulfinsäure, | 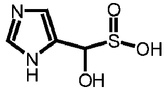 |
| Hydroxy(1-methyl-5-nitro-1*H*-imidazol-2-yl)methansulfinsäure, | 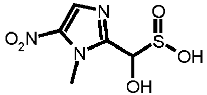 |
| Hydroxy(1,3-thiazol-2-yl)methansulfinsäure, | 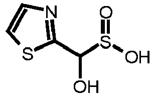 |
| Hydroxy(1,3-oxazol-2-yl)methansulfinsäure, | 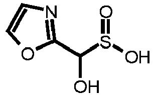 |
| Hydroxy(1*H*-pyrrol-2-yl)methansulfinsäure, | 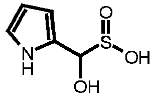 |
| Hydroxy(hydroxyl-2-yl)methansulfinsäure, | 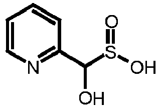 |
| Hydroxy(hydroxyl-4-yl)methansulfinsäure, | 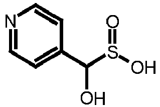 |
| Hydroxy[3-hydroxy-5-(hydroxymethyl)-2-methylpyridin-4-yl]-methansulfinsäure, | 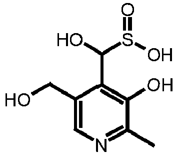 |
| Hydroxy(chinolin-2-yl)methansulfinsäure, | 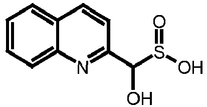 |
| Hydroxy(chinolin-4-yl)methansulfinsäure, | 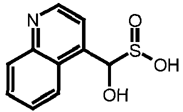 |
| 1*H*-Benzimidazol-2-yl(hydroxyl)methansulfinsäure, | 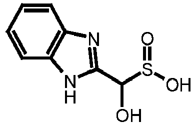 |
| 1,3-Benzothiazol-2-yl(hydroxyl)methansulfinsäure, | 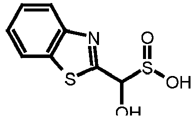 |
| 1,3-Benzoxazol-2-yl(hydroxy)methansulfinsäure, | 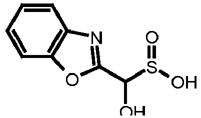 |
| Hydroxy{5-[hydroxy(sulfino)methyl]thien-2-yl}methansulfinsäure, | 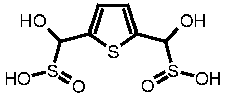 |
| 4-Hydroxy-4-sulfinobutansäure, | 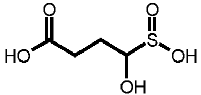 |
| 1-Hydroxy-4-methoxy-4-oxobutan-1-sulfinsäure, | 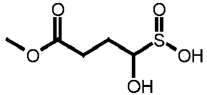 |
| 1-Hydroxy-4-ethoxy-4-oxobutan-1-sulfinsäure, | 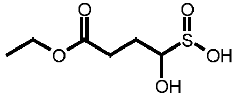 |
| 4-Hydroxy-4-sulfinopentansäure, | 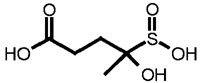 |
| 2-Hydroxy-5-methoxy-5-oxopentan-2-sulfinsäure, | 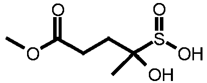 |
| 2-Hydroxy-5-ethoxy-5-oxopentan-2-sulfinsäure, | 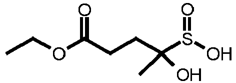 |
| 5-Hydroxy-5-sulfinopentansäure, | 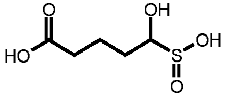 |
| 1-Hydroxy-5-methoxy-5-oxopentan-1-sulfinsäure, | 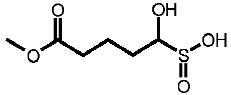 |
| 1-Hydroxy-5-ethoxy-5-oxopentan-1-sulfinsäure, | 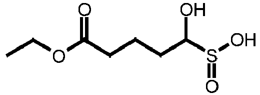 |
| 5-Hydroxy-5-sulfinohexansäure, | 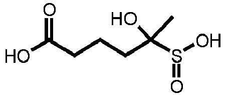 |
| 2-Hydroxy-6-methoxy-6-oxohexan-2-sulfinsäure, | 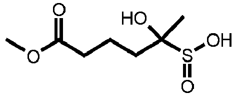 |
| 2-Hydroxy-6-ethoxy-6-oxohexan-2-sulfinsäure, | 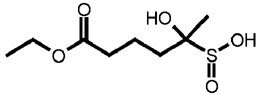 |
| 2-Hydroxy-2-sulfino-essigsäure | 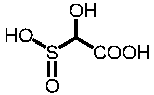 |
| 2-Amino-2-sulfino-essigsäure | 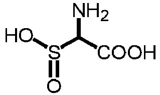 |
| Salz des 4-Hydroxy-1,1-dimethyl-4-sulfinopiperidiniums mit An- als Äquivalent eines ein- oder mehrwertigen Anions, | 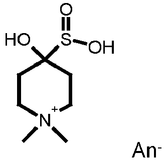 |
| Salz des 1-Allyl-4-hydroxy-1-methyl-4-sulfinopiperidiniums mit An- als Äquivalent eines ein- oder mehrwertigen Anions, | 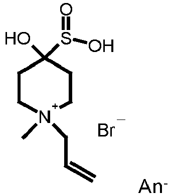 |
| 2-Hydroxy-2-sulfino-propionsäure | 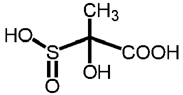 |
| 2-Amino-2-sulfino-propionsäure | 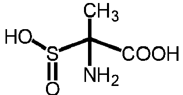 |
| 4-Hydroxy-1,1-dimethylpiperidinium-4-sulfinat, | 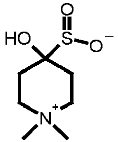 |
| 1-Allyl-4-hydroxy-1-methylpiperidinium-4-sulfinat, | 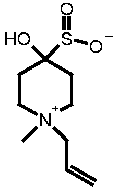 |
| 3-Hydroxy-3-sulfinato-propionsäure | 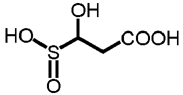 |
| 1-Hydroxy-2-(trimethylammonio)ethansulfinat, | 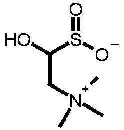 |
| 3-Amino-3-sulfinato-propionsäure | 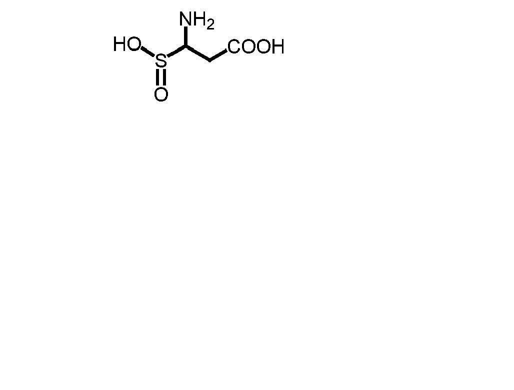 |

Für die bevorzugten ein- oder mehrwertigen Kationen aller Salze der zuvor genannten Verbindungen gilt das zuvor Gesagte.

Die Herstellung der erfindungsgemäß eingesetzten Sulfinsäuren der Formel (I) erfolgt beispielsweise aus der Umsetzung von Dithionit mit entsprechenden Aldehyden bzw. Ketonen analog zu der Herstellvorschrift gemäß WO-A1 -99/18067.

Bevorzugt enthält das erfindungsgemäße Mittel mindestens ein Sulfinsäurederivat der Formel (I) in einer Menge von 0,01 bis 20 Gew.-%, besonders bevorzugt von 1 bis 20 Gew.-%, jeweils bezogen auf das Gewicht des anwendungsbereiten Mittels.

Unter Verbindungen, besitzend mindestens ein quartäres Stickstoffatom, verstehen sich im Sinne der Erfindung alle chemischen Verbindungen, die mindestens ein quartäres, kationisches Stickstoffatom tragen. Diese werden auch als quartäre Ammoniumverbindungen bezeichnet. Das quartäre Stickstoffatom ist vierbindig. Somit sind quartäre Ammoniumreste und quartäre Iminiumreste umfasst. Die an das quartäre Stickstoffatom bindenden Gruppen sind allesamt von Wasserstoff verschieden und bilden mindestens drei Bindungen zu Kohlenstoffatomen organischer Reste aus.

Besagte Verbindungen, besitzend mindestens ein quartäres Stickstoffatom, sind von den Sulfinsäuren der Formel (I) verschieden.

Die erfindungsgemäßen Mittel enthalten mindestens eine Verbindung, besitzend mindestens ein quartäres Stickstoffatom, ausgewählt aus der Gruppe, die gebildet wird aus Verbindungen mit einem quartären Stickstoffatom. Die Verbindungen mit quartärem Stickstoffatom sind netto kationisch geladen. Die besagten Verbindungen liegen folglich nicht als ungeladenes Zwitterion oder gar anionisch vor.

Die Verbindungen, besitzend mindestens ein quartäres Stickstoffatom sind in dem erfindungsgemäßen Mittel bevorzugt in einer Menge von 0,01 Gew.-% bis 20 Gew.-%, besonders bevorzugt von 0,1 Gew.-% bis 5 Gew.-%, jeweils bezogen auf das Gewicht des anwendungsbereiten Mittels, enthalten.

Erfindungsgemäße quaternierte Ammoniumverbindungen werden ausgewählt aus Verbindungen mit der Formel (Q1-c),

(R¹⁰)_{y}(Me)_{(4-y)}N⁺X⁻ (Q1-c)

wobei
R¹⁰ unabhängig voneinander eine (C₈- bis C₃₀)-Alkylgruppe,
y gleich 1 oder 2 ist und
X⁻ ein physiologisch verträgliches organisches oder anorganisches Anion ist,

In den Verbindungen der Formel (Q1-c)steht der Rest R¹⁰ bevorzugt für eine Alkylgruppe mit 10 bis 22 Kohlenstoffatomen, besonders bevorzugt mit 10 bis 18 Kohlenstoffatomen.
Die in den erfindungsgemäßen Mitteln als Verbindungen der Formel (Q1-c) enthaltenen quartären Ammoniumverbindungen sind bevorzugt Halogenide, insbesondere Chloride und Bromide.

Beispiele für eine (C₈- bis C₃₀)-Alkylgruppe sind n-Octyl, n-Decyl, n-Dodecyl, n-Tetradecyl, n-Hexadecyl, Stearyl, Behenyl, Isostearyl und Eicosyl.
Als physiologisch verträgliches organisches oder anorganisches Gegenionen X⁻gemäß Formel (Q1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfat und Halogenidionen, insbesondere Bromid und Chlorid.

Die Verbindungen der Formel (Q1-c) werden bevorzugt ausgewählt aus der Gruppe, die gebildet wird aus
- Salze, insbesondere Halogenide, des Dicetyldimethylammoniums, Distearyldimethylammoniums (z.B. Dioctadecyldimethylammonium Chlorid als Genamin^{®} DSAc der Firma Clariant), Cetyltrimethylammoniums (z.B. Hexadecyltrimethylammonium Chlorid als Dehyquart^{®} A der Firma Cognis), oder des Stearyltrimethylammoniums.

Zusätzlich zu der erfindungsgemäßen Wirkstoffkombination können die erfindungsgemäßen Mittel als weitere Komponente mindestens eine Verbindung aus den Aldehyden und/oder den Ketonen enthalten.

Der Auswahl der Verbindungen aus den Aldehyden bzw. den Ketonen ist zunächst keine Grenze gesetzt. Als besonders geeignet erweist sich mindestens ein Vertreter aus der Gruppe:
- Oxocarbonsäuren oder deren Salze,
- Oxocarbonsäureester,
- cyclische, lineare oder verzweigte aliphatische Aldehyde,
- cyclische, lineare oder verzweigte aliphatische Ketone,
- mono- bis polyhydroxyfunktionalisierte Aldehyde,
- mono- bis polyhydroxyfunktionalisierte Ketone,
- alicyclische, aromatische oder heterocyclische Aldehyde sowie
- alicyclische, aromatische oder heterocyclische Ketone.

Generell gilt, dass in den oben genannten Verbindungen der cyclischen, alicyclischen bzw. heterocyclischen Ketone die Ketogruppe(n) endocyclisch (und/oder) oder exocyclisch gebunden sein kann (können).

Oxocarbonsäuren sind organische Verbindungen, die neben mindestens einer Carboxylgruppe eine Carbonylgruppe tragen und sind somit Aldehyd- bzw. Ketocarbonsäuren. Bevorzugte Oxocarbonsäuren sind α-Oxocarbonsäuren, β-Oxocarbonsäuren, γ-Oxocarbonsäuren sowie ω-Oxocarbonsäuren der Formel (XII) bzw. deren Salze, 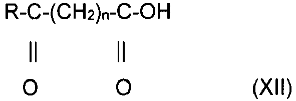 worin bedeuten
- R: ein Wasserstoffatom, eine (C₁ bis C₆)-Alkylgruppe, eine (C₁ bis C₆)-Hydroxyalkylgruppe, eine gegebenenfalls substituierte Arylgruppe, eine gegebenenfalls substituierte Heteroarylgruppe, eine (C₂ bis C₆)-Alkenylgruppe oder eine Carboxy-(C₁ bis C₆)-alkylgruppe,
- n: eine Zahl 0, 1, 2 oder 3.
Besonders bevorzugt werden die Oxocarbonsäuren ausgewählt aus mindestens einem Vertreter aus der Gruppe Glyoxalsäure, Acetessigsäure, 3-Oxoglutarsäure, 4-Oxovaleriansäure und Brenztraubensäure bzw. den Salzen der vorgenannten Säuren.

Die Oxocarbonsäureester werden bevorzugt ausgewählt aus (C₁ bis C₆)-Alkylestern der erfindungsgemäß bevorzugten Oxocarbonsäuren.

Unter cyclischen, linearen oder verzweigten aliphatischen Aldehyden sind erfindungsgemäß aliphatische Aldehyde zu verstehen, deren Formylgruppe(n) nicht in Konjugation mit einem aromatischen π-Elektronen-System stehen. Die entsprechenden Aldehyde dürfen aromatische Reste tragen, solange die π-Elektronen der Formylgruppe(n) nicht über ein solches aromatisches System delokalisiert sein können. Bevorzugte cyclische, lineare oder verzweigte aliphatische Aldehyde sind gesättigt oder ungesättigt und werden besonders bevorzugt ausgewählt aus mindestens einem Vertreter aus der Gruppe Formaldehyd, Acetaldehyd, Glyoxal, Propionaldehyd, Butanal, Pentanal, Isopentanal, Hexanal, Cyclohexanal, Heptanal, Octanal, Malondialdehyd, Glutaraldehyd, 2-Methylpentanal, 2-Ethylhexanal, 3,5,5-Trimethylhexanal, 2-Ethylbutyraldehyd, 2-Methylbutyraldehyd, Isobutyraldehyd, 3-Phenylpropanal, 3-(4-Methylphenyl)propanal, 3-(4-Methoxyphenyl)propanal, 3-(2-Methoxyphenyl)propanal, 2-Butenal, Acrolein, 3-Methyl-2-butenal, 3,7-Dimethyl-2,6-octadienal, 2,4-Pentadienal, 3,7-Dimethyl-6-octenal, 2,4-Dimethyl-3-cyclohexencarboxaldehyd und 2,6-Nonadienal.

Unter cyclischen, linearen oder verzweigten aliphatischen Ketonen sind erfindungsgemäß aliphatische Ketone zu verstehen, deren Ketogruppe(n) nicht in Konjugation mit einem aromatischen π-Elektronen-System stehen. Die entsprechenden Ketone dürfen jedoch aromatische Reste tragen, solange die π-Elektronen der Ketogruppe(n) nicht über ein solches aromatisches System delokalisiert sein können. Bevorzugte cyclische, lineare oder verzweigte aliphatische Ketone sind gesättigt oder ungesättigt und ausgewählt aus mindestens einem Vertreter aus der Gruppe Aceton, 2-Butanon, 2-Pentanon, 3-Pentanon, 2-Hexanon, 3-Hexanon, Butan-2,4-dion, Pentan-2,4-dion, Hexan-2,5-dion, Cyclohexanon, Cyclopentanon, 4-Methylpentan-2-on, 5-Methyl-3-hexen-2-on, 2-(3-Oxopropyl)benzoesäuremethylester und 4-(3-Oxopropyl)benzoesäuremethylester.

Bevorzugt verwendbare monohydroxyfunktionalisierte Aldehyde werden ausgewählt aus mindestens einem Vertreter aus der Gruppe 1-Hydroxypropanal, 5-Hydroxypentanal, 3,7-Dimethyl-7-hydroxyoctanal, Hydroxyisohexyl-3-cyclohexen-1-carboxaldehyd und 2,6,6-Trimethyl-1,3-cyclohexadien-1-carboxaldehyd.

Polyhydroxyfunktionalisierte Aldehyde sind erfindungsgemäß bevorzugt die sogenannten Aldosen und werden besonders bevorzugt ausgewählt aus mindestens einem Vertreter aus der Gruppe 2,3-Dihydroxypropionaldehyd, D-Erythrose, D-Threose, D-Ribose, D-Arabinose, D-Lyxose, D-Xylose, D-Allose, D-Altrose, D-Galactose, D-Glucose, D-Idose, D-Mannose, D-Rhamnose und D-Talose, sowie den L-Konfigurationen L-Erythrose, L-Threose, L-Ribose, L-Arabinose, L-Lyxose, L-Xylose, L-Allose, L-Altrose, L-Galactose, L-Glucose, L-Idose, L-Mannose, L-Rhamnose und L-Talose. Monohydroxyfunktionalisierte Ketone, die sich erfindungsgemäß besonders eignen, werden bevorzugt ausgewählt aus mindestens einem Vertreter aus der Gruppe 1-Hydroxy-2-propanon, 1-Hydroxy-2-butanon, 3-Hydroxy-2-butanon und Benzoin.

Polyhydroxyfunktionalisierte Ketone sind erfindungsgemäß bevorzugt die sogenannten Ketosen und werden besonders bevorzugt ausgewählt aus mindestens einem Vertreter aus der Gruppe 1,3-Dihydroxyaceton, D-Psicose, D-Fructose, D-Sorbose, D-Tagatose, D-Ribulose, D-Xylulose, D-Erythrulose sowie den L-Konfigurationen L-Psicose, L-Fructose, L-Sorbose, L-Tagatose, L-Ribulose, L-Xylulose, L-Erythrulose.

Bevorzugte alicyclische, aromatische oder heterocyclische Ketone werden bevorzugt ausgewählt aus mindestens einem Vertreter aus der Gruppe
- Benzylidenketone, insbesondere den Benzylidenketonen aus der Patentanmeldung DE-A1-19717281 (siehe DE-A1-19717281: Formel (I) des 1. Anspruchs und die Vertreter gemäß 4. Anspruch), auf die ausdrücklich Bezug genommen wird,
- Imidazolin-2,4-dion und dessen Derivaten, insbesondere Allantoin und/oder 5,5-Dimethylhydantoin.
Bevorzugte alicyclische, aromatische oder heterocyclische Aldehyde werden bevorzugt ausgewählt aus mindestens einem Vertreter aus der Gruppe
- Benzaldehyd und seinen Derivaten,
- Zimtaldehyd und seinen Derivaten sowie
- Naphthaldehyd und seinen Derivaten.

Alle Aldehyd- bzw. Ketoverbindungen sollen aus physiologisch verträglichen bzw. nicht toxischen Verbindungen ausgewählt werden, wenn das erfindungsgemäße Mittel als kosmetisches Mittel Verwendung finden soll.

Die Verbindungen, ausgewählt aus Aldehyden oder Ketonen, sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Es kann erfindungsgemäß bevorzugt sein, das erfindungsgemäße Mittel mit der Maßgabe bereitzustellen, dass die in dem erfindungsgemäßen Mittel eingesetzten Verbindungen, ausgewählt aus den Aldehyden und/oder den Ketonen, sich von denjenigen Aldehyden bzw. Ketonen unterscheiden müssen, von denen sich diejenigen Sulfinsäuren ableiten, die in dem erfindungsgemäßen Mittel tatsächlich enthalten sind.

Es ist ebenso erfindungsgemäß bevorzugt, wenn das erfindungsgemäße Mittel zusätzlich mindestens ein Redukton enthält. Unter einem Redukton versteht der Fachmann reduktiv wirkende Endiol-Verbindungen, die durch Substitution in α-Stellung stabilisiert sind und die der Tautomerie unterliegen. Die wichtigsten erfindungsgemäß einsetzbaren Reduktone sind Ascorbinsäure, Isoascorbinsäure, 2,3-Dihydroxy-2-propendial und 2,3-Dihydroxy-2-cyclopentenon.
Die Reduktone sind erfindungsgemäß bevorzugt in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, in dem erfindungsgemäßen Mittel enthalten.

Als Träger für das erfindungsgemäße Mittel eignen sich bevorzugt flüssige Medien, in denen das erfindungsgemäße Sulfinsäurederivat bevorzugt löslich ist, wie beispielsweise Wasser oder organische Lösemittel. Es ist erfindungsgemäß bevorzugt, wenn der Träger ein kosmetischer Träger ist.

Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Weitere alkoholische Lösemittel, sind beispielsweise Methoxybutanol, Benzylalkohol, 2-Phenoxyethanol, Ethyldiglykol oder 1,2-Propylenglykol. In einer bevorzugten Ausführungsform enthält das erfindungsgemäße Mittel zusätzlich als Lösemittel mindestens einen (C₂ bis C₆)-Alkylmonoalkohol und/oder ein (C₂ bis C₆)-Alkandiol, insbesondere Ethanol, Isopropanol und/oder 1,2-Propylenglykol.

Das erfindungsgemäße Mittel besitzt bevorzugt einen pH-Wert von pH 1 bis pH 9, insbesondere von pH 1,5 bis pH 6.

Ein zweiter Gegenstand der Erfindung ist die Verwendung des Mittels gemäß erstem Erfindungsgegenstand zur Entfärbung von Substraten, die mit natürlichen und/oder synthetischen Farbstoffen eingefärbt sind.

Das Substrat enthält natürliche Fasern.

Die natürlichen Fasern werden ausgewählt aus keratinhaltigen Fasern, insbesondere Wolle oder tierischen oder menschlichen Haaren, ganz besonders bevorzugt aus menschlichen Haaren.

Die zu entfärbenden Substrate sind bevorzugt mit Oxidationsfarbstoffen und/oder direktziehenden Farbstoffen, als Vertreter der synthetischen Farbstoffe, gefärbt.

In einer weiteren bevorzugten Ausführungsform wurden die zu entfärbenden Substrate mit einem oxidativen Färbemittel gefärbt, welches neben mindestens einer Entwicklerkomponente zusätzlich mindestens eine Kupplerkomponente enthält.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können die zu entfärbenden Substrate mit Vorstufen naturanaloger Farbstoffe bevorzugt unter Zuhilfenahme solcher Indole und Indoline gefärbt sein, die bevorzugt mindestens zwei Hydroxy- oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin. Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol. Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol. Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die zu entfärbenden Substrate bevorzugt mit einem kationischen direktziehenden Farbstoff gefärbt sein. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Ein dritter Gegenstand der Erfindung ist ein Verfahren zur reduktiven Entfärbung von mit natürlichen und/oder synthetischen Farbstoffen eingefärbten Substraten, wobei die Substrate natürliche Fasern, ausgewählt aus keratinhaltigen Fasern, enthalten, in dem ein Mittel des ersten Erfindungsgegenstandes auf das eingefärbte Substrat aufgetragen wird und nach einer Einwirkzeit wieder abgespült wird.

Die Einwirkzeit beträgt bevorzugt 1 bis 60 Minuten, bevorzugt 5 bis 30 Minuten. Die Einwirkung des erfindungsgemäßen Mittels kann nicht nur bei Raumtemperatur, sondern bevorzugt in einem Temperaturbereich von 15 bis 60 °C, insbesondere von 25 bis 60 °C erfolgen.

Nach Ablauf der Einwirkzeit werden die Haare ausgespült, wobei bevorzugt ein tensidhaltiges Mittel, wie beispielsweise ein Reinigungsmittel oder ein Shampoo, Anwendung findet. Gegebenenfalls kann das Substrat mehrfach ausgespült, bzw. mehrfach mit dem tensidhaltigen Mittel behandelt werden.

Das tensidhaltige Mittel zur Spülung bzw. Nachbehandlung enthält bevorzugt in einem Träger mindestens ein Tensid, ausgewählt aus anionischen, zwitterionischen, ampholytischen, nichtionischen oder kationischen Tensiden, insbesondere aus nichtionischen Tensiden.

Als anionische Tenside eignen sich in den erfindungsgemäßen tensidhaltigen Mitteln zur Spülung bzw. Nachbehandlung alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für die in den erfindungsgemäßen tensidhaltigen Mitteln zur Spülung bzw. Nachbehandlung verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methyl-hydroxyalkyldialkoyloxyalkylammoniummethosulfate.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Die Tenside werden bevorzugt in Konzentrationen von 0,5 bis 30 Gew.-%, bezogen auf das Gewicht der Zusammensetzung, in dem tensidhaltigen Mittel zur Spülung bzw. Nachbehandlung eingesetzt.

Zusätzlich besitzt das tensidhaltige Mittel zur Spülung bzw. Nachbehandlung einen pH-Wert von kleiner pH 7. Dieser pH-Wert wird bevorzugt durch den Zusatz eines Puffers während des Spül- bzw. Nachbehandlungsschritts konstant gehalten. Erfindungsgemäß bevorzugt geeignete Puffersysteme sind der Phosphatpuffer (PO₄³⁻ / HPO₄²⁻ / H₂PO₄⁻ / H₃PO₄), Essigsäure/Acetat-Puffer, Citronensäure/Citrat-Puffer, KCl / HCl-Puffer, Monophthalat/HCl-Puffer oder Monophthalat/NaOH-Puffer.

Nach dem Ausspülen kann es vorteilhaft sein, das Substrat mit einer Oxidationsmittelhaltigen Zusammensetzung zu behandeln. Bevorzugt wird Wasserstoffperoxid als Oxidationsmittel, bevorzugt in Konzentrationen von 0,5 bis 6 Gew.-%, eingesetzt. Die Einwirkzeit beträgt bevorzugt 1 bis 30 Minuten, besonders bevorzugt 1 bis 10 Minuten. Nach Ablauf der Einwirkzeit wird die Oxidationsmittelhaltige Zusammensetzug ausgespült.

Die eingefärbten Substrate sind dieselben, die im zweiten Erfindungsgegenstand definiert wurden.

Als bevorzugte natürliche oder synthetische Farbstoffe wurden bevorzugt diejenigen Farbstoffe verwendet, wir sie im zweiten Erfindungsgegenstand definiert wurden.

Der Erfindungsgegenstand soll exemplarisch anhand der folgenden Ausführungen erläutert werden.

### Beispiele

Es wurden die erfindungsgemäßen Entfärbemittel gemäß Tabelle 1 bereitgestellt. Dabei wurden folgende Rohstoffe verwendet:

| Rohstoff: | Inhalt | Hersteller: |
|---|---|---|
| Aromox^{®} MCDW | Dimethyl(kokosnussölalkyl)aminoxid; 30 Gew.- % Aktivsubstanz in Wasser (INCI-Bezeichnung: Cocamine Oxide) | Akzo Nobel |
| Hostapur^{®} OS | C₁₅₋₁₈-Olefinsulfonat, Natriumsalz (Pulver) | Clariant |
| Brüggolit^{®} FF7 | enthält ca. 65 % 2-Hydroxy-2-sulfinoessigsäure Natriumsalz | Brüggemann |

Die Rohstoffe wurden nach und nach in 75 Gew.-% Wasser unter Rühren gemischt. Anschließend wurde der pH-Wert eingestellt und ggf. mit Wasser auf 100 Gew.-% aufgefüllt.

**Tabelle 1: Entfärbemittel**

| Rohstoff | E1¹⁾ | E2 |
|---|---|---|
| | [Gew.-%] | [Gew.-%] |
| Brüggolit^{®} FF7 | 10,0 | 10,0 |
| Aromox^{®} MCDW | 3,0 | - |
| Cetyltrimethylammoniumchlorid | - | 3,0 |
| H₃PO₄ | ad pH 1,5 | ad pH 1,5 |
| Wasser | ad 100 | ad 100 |
| ¹⁾ nicht erfindungsgemäß | | |

### Verfahren 1:

Folgende Prozedur wurde für alle Entfärbemittel der Tabelle 1 durchgeführt:
Eine Haarsträhne (Naturhaar, Fa. Kerling) wurde mit einer handelsüblichen oxidativen Haarfarbe (Igora^{®} Royal (Schwarzkopf), Färbecreme Naturton im Gewichtsverhältnis 1 zu 1 mit dem Entwickler Oxigenta^{®} (6 Gew.-% Wasserstoffperoxid, Fa. Schwarzkopf) über eine Einwirkzeit von 30 Minuten bei Raumtemperatur gefärbt, gespült und für 24 Stunden gelagert. Danach wurde die Strähne in 30 mL eines Entfärbemittels der Tabelle 1 über eine Einwirkzeit von 30 Minuten bei Raumtemperatur getaucht, anschließend mit viel Wasser gespült und an der Luft getrocknet.

Alle Haarsträhnen zeigten nach der Entfärbeprozedur eine hervorragende Aufhellung und eine geringe Haarschädigung. Die Haarsträhnen erfuhren auch nach 2 Wochen Lagerung keine Nachdunkelung.

### Verfahren 2:

Folgende Prozedur wurde für alle Entfärbemittel der Tabelle 1 durchgeführt:
Es wurde eine mit der oxidativen Haarfarbe gemäß Versuch 1 gefärbte Haarsträhne (Naturhaar, Fa. Kerling) in 30 mL eines der Entfärbemittel der Tabelle 1 über eine Einwirkzeit von 30 Minuten bei Raumtemperatur getaucht. Abweichend zum Versuch 1 wurde die Strähne jedoch anschließend für 2 Minuten in 50 mL eines der tensidhaltigen Mittel zur Spülung gemäß Tabelle 2 gewaschen, für weniger als 1 Minute unter fließendem Wasser gespült und an der Luft getrocknet.

**Tabelle 2: tensidhaltige Mittel zur Spülung**

| Rohstoff | B1 | B2 | B3 |
|---|---|---|---|
| | [Gew.-%] | [Gew.-%] | [Gew.-%] |
| KH₂PO₄ | 5,0 | 5,0 | 5,0 |
| Aromox^{®} MCDW | 10,0 | - | - |
| Cetyltrimethylammoniumchlorid | - | 10,0 | - |
| Hostapur^{®} OS | - | - | 10,0 |
| H₃PO₄ | ad pH 2 | ad pH 2 | ad pH 2 |
| Wasser | ad 100 | ad 100 | ad 100 |

Alle Haarsträhnen zeigten mit jedem der Mittel B1 bis B3 nach der Spülung eine verbesserte Aufhellung im Vergleich zu einer Haarsträhne aus Verfahren 1, die lediglich mit Wasser gespült wurde. Die Haarschädigung ist gering. Die Haarsträhnen erfuhren auch nach 2 Wochen Lagerung keine Nachdunkelung.

## Patentansprüche

1. Mittel zum reduktiven Farbabzug keratinischer Fasern, enthaltend in einem Träger mindestens ein Sulfinsäurederivat der Formel (I) 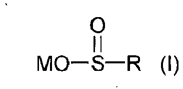 worin
M steht für ein Wasserstoffatom oder ein Äquivalent eines ein- oder mehrwertigen Kations,
R für einen Rest gemäß der Formel (II) steht, 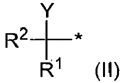
worin bedeuten
Y unabhängig voneinander eine Hydroxygruppe, eine -NH₂ Gruppe oder eine Gruppe -NR³R⁴, wobei R³ und R⁴ unabhängig voneinander für eine (C₁ bis C₆)-Alkylgruppe, eine (C₂ bis C₆)-Alkenylgruppe, eine (C₁ bis C₆)-Hydroxyalkylgruppe, eine (C₂ bis C₆)-Polyhydroxyalkylgruppe, eine Arylgruppe oder eine Aryl-(C₁ bis C₆)-alkylgruppe stehen,
R¹ unabhängig voneinander ein Wasserstoffatom, eine (C₁ bis C₆)-Alkylgruppe oder eine Carboxyalkylgruppe -(CH₂)ₘ-COOM^{II}, in der M" für ein Wasserstoffatom oder für ein Äquivalent eines ein- oder mehrwertigen Kations steht und m die Zahl 0, 1 oder 2 bedeutet,
R² ein Wasserstoffatom, eine (C₁ bis C₆)-Alkylgruppe, einen Carboxyalkylrest -(CH₂)ₙ-COOM^{III} mit
M^{III} = Wasserstoffatom oder ein Äquivalent eines ein- oder mehrwertigen Kations und n eine ganze Zahl 0, 1, 2, 3, 4, 5 oder 6, einen (C₁ bis C₆)-Alkyloxycarbonylrest, eine N,N,N-Tri[(C₁ bis C₆)-alkyl]ammonium-(C₁ bis C₆)-alkylgruppe eine Carboxy-(C₂ bis C₆)-alkenylgruppe, eine Cyano-(C₁ bis C₆)-alkylgruppe oder eine (C₁ bis C₆)-Alkoxycarbonyl-(C₁ bis C₆)-alkylgruppe, oder
R² zusammen mit R¹ und dem Restmolekül einen aliphatischen 5-, 6-, oder 7-gliedrigen Ring bildet, welcher mindestens ein kationisches, quaterniertes Stickstoffatom als Heteroatom enthält, wobei die kationische Ladung gegebenenfalls durch ein Äquivalent eines ein- oder mehrwertigen Anions kompensiert wird, oder
einen aliphatischen oder aromatischen Heterozyklus, der substituiert sein kann,
mit der Maßgabe, dass in Formel (II) R¹ und R² nicht gleichzeitig für ein Wasserstoffatom stehen,
in Kombination mit mindestens einer Verbindung, besitzend mindestens ein quartäres Stickstoffatom, wobei die Verbindungen, besitzend mindestens ein quartäres Stickstoffatom ausgewählt werden aus Verbindungen mit der Formel (Q1-c),
(R¹⁰)_{y}(Me)_{(4-y)}N⁺ X⁻ (Q1-c)
worin
- R¹⁰ unabhängig voneinander eine (C₈- bis C₃₀)-Alkylgruppe bedeutet,
- y gleich 1 oder 2 ist und
- X- ein physiologisch verträgliches organisches oder anorganisches Anion ist.

2. Mittel nach Anspruch 1, **dadurch gekennzeichnet, daß** in der Formel (I) M, M^{II} und M^{III} unabhängig voneinander stehen für ein Wasserstoffatom, ein Ammoniumion, ein Alkalimetallion, für ein halbes Äquivalent eines Erdalkalimetallions oder ein halbes Äquivalent eines Zinkions, insbesondere für ein Wasserstoffatom, ein Ammoniumion, ein Natriumion, ein Kaliumion, ½ Kalziumion, ½ Magnesiumion oder ½ Zinkion.

3. Mittel nach einem der Ansprüche 1 oder 2, **dadurch gekennzeichnet, daß** gemäß Formel (I) die Reste Y der Formel (II) unabhängig voneinander eine Hydroxygruppe oder eine Gruppe-NH₂ bedeuten.

4. Mittel nach einem der Ansprüche 1 bis 3, **dadurch gekennzeichnet, daß** es das Sulfinsäurederivat in einer Menge von 0,01 bis 20 Gew.-%, besonders bevorzugt von 1 bis 20 Gew.-%, jeweils bezogen auf das Gewicht des Mittels, enthält.

5. Mittel nach einem der Ansprüche 1 bis 4, **dadurch gekennzeichnet, daß** es zusätzlich Verbindungen aus den Aldehyden bzw. den Ketonen enthält, ausgewählt aus mindestens einem Vertreter aus der Gruppe
- Oxocarbonsäuren oder deren Salze,
- Oxocarbonsäureester,
- cyclische, lineare oder verzweigte aliphatische Aldehyde,
- cyclische, lineare oder verzweigte aliphatische Ketone,
- mono- bis polyhydroxyfunktionalisierte Aldehyde,
- mono- bis polyhydroxyfunktionalisierte Ketone,
- alicyclische, aromatische oder heterocyclische Ketone sowie
- alicyclische, aromatische oder heterocyclische Aldehyde.

6. Mittel nach Anspruch 5, **dadurch gekennzeichnet, daß** die Verbindungen, ausgewählt aus Aldehyden oder Ketonen, in einer Menge von 0,1 Gew.-% bis 20 Gew.-%, insbesondere von 0,5 Gew.-% bis 10 Gew.-%, jeweils bezogen auf das Gewicht des anwendungsbereiten Mittels, enthalten sind.

7. Mittel nach einem der Ansprüche 1 bis 6, **dadurch gekennzeichnet, daß** es einen pH-Wert von pH 1 bis pH 9, insbesondere von pH 1,5 bis pH 6, besitzt.

8. Mittel nach einem der Ansprüche 1 bis 7, **dadurch gekennzeichnet, daß** es zusätzlich als Lösemittel mindestens einen (C₂ bis C₆)-Alkylmonoalkohol und/oder (C₂ bis C₆)-Alkandiol enthält, insbesondere Ethanol, Isopropanol und/oder 1,2-Propylenglykol.

9. Verwendung eines Mittels gemäß einem der Ansprüche 1 bis 8 zur Entfärbung von Substraten, die mit Oxidationsfarbstoffen und/oder direktziehenden Farbstoffen gefärbt sind, wobei die Substrate natürliche Fasern, ausgewählt aus keratinhaltigen Fasern, enthalten.

10. Verfahren zur reduktiven Entfärbung von mit natürlichen und/oder synthetischen Farbstoffen eingefärbten Substraten, wobei die Substrate natürliche Fasern, ausgewählt aus keratinhaltigen Fasern, enthalten, **dadurch gekennzeichnet, daß** ein Mittel gemäß einem der Ansprüche 1 bis 8 auf das eingefärbte Substrat aufgetragen wird und nach einer Einwirkzeit wieder abgespült wird.

## Claims

1. Agent for the reductive color removal of keratin fibers, containing in a carrier at least one sulfinic acid derivative of the formula (I) 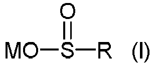 wherein
M represents a hydrogen atom or an equivalent of a monovalent or polyvalent cation,
R represents a radical according to the formula (II), 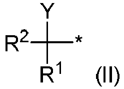
with
Y independently represents a hydroxy group, a -NH2 group or a group -NR3R4, where R3 and R4 independently represent a (C1 to C6) alkyl group, a (C2 to C6) alkenyl group, a (C1 to C6) hydroxyalkyl group, a (C2 to C6) polyhydroxyalkyl group, an aryl group or an aryl (C1 to C6) alkyl group,
R¹ independently represents a hydrogen atom, a (C1 to C6) alkyl group or a carboxyalkyl group -(CH2)m-COOM", in which M^{II} represents a hydrogen atom or an equivalent of a monovalent or polyvalent cation and m is the number 0, 1 or 2 means
R² is a hydrogen atom, a (C1 to C6) alkyl group,
a carboxyalkyl radical -(CH₂)ₙ-COOM^{III}
M^{III} = hydrogen atom or an equivalent of a monovalent or polyvalent cation and n is an integer 0, 1, 2, 3, 4, 5 or 6,
a (C1 to C6)-alkyloxycarbonyl radical, an N,N,N-tri[(C1 to C6)-alkyl]ammonium-(C1 to C6)-alkyl group, a carboxy-(C2 to C6)-alkenyl group, a cyano-( C1 to C6) alkyl group or a (C1 to C6) alkoxycarbonyl (C1 to C6) alkyl group, or
R² together with R¹ and the remaining molecule forms an aliphatic 5-, 6- or 7-membered ring which contains at least one cationic, quaternized nitrogen atom as a heteroatom, the cationic charge being optionally compensated for by an equivalent of a monovalent or polyvalent anion, or an aliphatic or aromatic heterocycle, which can be substituted,
with the proviso that in formula (II) R¹ and R² do not simultaneously represent a hydrogen atom,
in combination with at least one compound having at least one quaternary nitrogen atom, the compounds having at least one quaternary nitrogen atom being selected from compounds with the formula (Q1-c),
(R¹⁰)_{y}(Me)(_{4-y})N⁺ X⁻ (Q1-c)
wherein
- R¹⁰ independently represents a (C8 to C30) alkyl group,
- y is equal to 1 or 2 and
- X- is a physiologically acceptable organic or inorganic anion.

2. Agent according to claim 1, **characterized in that** in the formula (I) M, M^{II} and M^{III} independently represent a hydrogen atom, an ammonium ion, an alkali metal ion, half an equivalent of an alkaline earth metal ion or half an equivalent of a zinc ion, in particular a hydrogen atom , one ammonium ion, one sodium ion, one potassium ion, ½ calcium ion, ½ magnesium ion or ½ zinc ion.

3. Agent according to one of claims 1 or 2, **characterized in that**, according to formula (I), the radicals Y of formula (II) independently represent a hydroxy group or a group -NH2.

4. Agent according to one of claims 1 to 3, **characterized in that** it contains the sulfinic acid derivative in an amount of 0.01 to 20% by weight, particularly preferably 1 to 20% by weight, based on the weight of the agent.

5. Agent according to one of claims 1 to 4, **characterized in that** it additionally contains compounds from the aldehydes or the ketones, selected from at least one representative of the group
- oxocarboxylic acids or their salts,
- oxocarboxylic acid esters,
- cyclic, linear or branched aliphatic aldehydes,
- cyclic, linear or branched aliphatic ketones,
- mono- to polyhydroxy-functionalized aldehydes,
- mono- to polyhydroxy-functionalized ketones,
- alicyclic, aromatic or heterocyclic ketones as well
- alicyclic, aromatic or heterocyclic aldehydes.

6. Composition according to claim 5, **characterized in that** the compounds, selected from aldehydes or ketones, in an amount of 0.1% by weight to 20% by weight, in particular from 0.5% by weight to 10% by weight. %, based on the weight of the ready-to-use agent, are included.

7. Agent according to one of claims 1 to 6, **characterized in that** it has a pH value of pH 1 to pH 9, in particular from pH 1.5 to pH 6.

8. Agent according to one of claims 1 to 7, **characterized in that** it additionally contains as a solvent at least one (C2 to C6) alkyl monoalcohol and / or (C2 to C6) alkanediol, in particular ethanol, isopropanol and / or 1,2 -propylene glycol.

9. Use of an agent according to one of claims 1 to 8 for the decolorization of substrates which are colored with oxidation dyes and/or direct dyes, the substrates containing natural fibers selected from keratin-containing fibers.

10. A process for the reductive decolorization of substrates colored with natural and/or synthetic dyes, the substrates containing natural fibers selected from keratin-containing fibers, **characterized in that** an agent according to one of claims 1 to 8 is applied to the colored substrate and rinsed off again after a period of exposure.

## Revendications

1. Agent décolorant réducteur des fibres kératiniques, contenant dans un support au moins un dérivé d'acide sulfinique de formule (I) 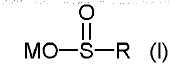 dans laquelle
M représente un atome d'hydrogène ou un équivalent d'un cation monovalent ou polyvalent,
R représente un radical de formule (II), 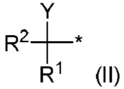
dans laquelle
Y représente indépendamment un groupe hydroxy, un groupe -NH2 ou un groupe -NR3R4, où R3 et R4 représentent indépendamment un groupe (C1 à C6) alkyle, un groupe (C2 à C6) alcényle, un groupe (C1 à C6) hydroxyalkyle, un groupe (C2 à C6) polyhydroxyalkyle, un groupe aryle ou un groupe aryl(C1 à C6)alkyle,
R1 représente indépendamment un atome d'hydrogène, un groupe alkyle (C1 à C6) ou un groupe carboxyalkyle
-(CH2)m-COOM", dans lequel M^{II} représente un atome d'hydrogène ou un équivalent d'un cation monovalent ou polyvalent et m est le nombre 0, 1 ou 2 signifie
R² est un atome d'hydrogène, un groupe alkyle (C1 à C6),
un radical carboxyalkyle -(CH2)n-COOM^{III}
M^{III} = atome d'hydrogène ou équivalent d'un cation monovalent ou polyvalent et n est un nombre entier 0, 1, 2, 3, 4, 5 ou 6,
un radical (C1 à C6)-alkyloxycarbonyle, un groupe N,N,N-tri[(C1 à C6)-alkyl]ammonium-(C1 à C6)-alkyle, un groupe carboxy-(C2 à C6)-alcényle, un groupe cyano-(C1 à C6) alkyle ou un groupe (C1 à C6) alcoxycarbonyl (C1 à C6) alkyle, ou
R² forme avec R¹ et la molécule restante un cycle aliphatique à 5, 6 ou 7 chaînons qui contient au moins un atome d'azote cationique quaternisé comme hétéroatome, la charge cationique étant éventuellement compensée par un équivalent d'un composé monovalent ou polyvalent anioniques, ou
un hétérocycle aliphatique ou aromatique, pouvant être substitué,
à condition que dans la formule (II) R¹ et R² ne représentent pas simultanément un atome d'hydrogène,
en combinaison avec au moins un composé ayant au moins un atome d'azote quaternaire, les composés ayant au moins un atome d'azote quaternaire étant choisis parmi les composés de formule (Q1-c),
(R¹⁰)_{y}(Me)(_{4-y})N⁺ X- (Q1-c)
dans laquelle
- R10 représente indépendamment un groupe alkyle (C8 à C30),
- y est égal à 1 ou 2 et
- X- est un anion organique ou inorganique physiologiquement acceptable.

2. Agent selon la revendication 1, **caractérisé en ce que** dans la formule (I) M, M^{II} et M^{III} représentent indépendamment un atome d'hydrogène, un ion ammonium, un ion de métal alcalin, un demi équivalent d'un ion alcalino-terreux ou un demi équivalent, d'un ion zinc, notamment un atome d'hydrogène, un ion ammonium, un ion sodium, un ion potassium, ½ ion calcium, ½ ion magnésium ou ½ ion zinc.

3. Agent selon l'une des revendications 1 ou 2, **caractérisé en ce que** selon la formule (I), les radicaux Y de formule (II) représentent indépendamment un groupe hydroxy ou un groupe -NH2.

4. Agent selon l'une des revendications 1 à 3, **caractérisé en ce qu'**il contient le dérivé de l'acide sulfinique à raison de 0,01 à 20 % en poids, de manière particulièrement préférée de 1 à 20 % en poids, par rapport au poids de l'agent.

5. Agent selon l'une des revendications 1 à 4, **caractérisé en ce qu'**il contient en outre des composés issus des aldéhydes ou des cétones, choisis parmi au moins un représentant du groupe
- les acides oxocarboxyliques ou leurs sels,
- les esters d'acide oxocarboxylique,
- les aldéhydes aliphatiques cycliques, linéaires ou ramifiés,
- les cétones aliphatiques cycliques, linéaires ou ramifiées,
- les aldéhydes fonctionnalisés mono- à polyhydroxy,
- les cétones mono- à polyhydroxyfonctionnalisées,
- également les cétones alicycliques, aromatiques ou hétérocycliques
- les aldéhydes alicycliques, aromatiques ou hétérocycliques.

6. Agent selon la revendication 5, **caractérisé en ce que** les composés, choisis parmi les aldéhydes ou les cétones, sont à raison de 0,1 % en poids à 20 % en poids, notamment de 0,5 % en poids à 10 % en poids, sur la base du poids de l'agent prêt à l'emploi, sont inclus.

7. Agent selon l'une des revendications 1 à 6, **caractérisé en ce qu'**il présente une valeur de pH de pH 1 à pH 9, notamment de pH 1,5 à pH 6.

8. Agent selon l'une des revendications 1 à 7, **caractérisé en ce qu'**il contient en outre comme solvant au moins un (C2 à C6) alkylmonoalcool et/ou (C2 à C6) alcanediol, notamment l'éthanol, l'isopropanol et/ou 1,2-propylèneglycol.

9. Utilisation d'un agent selon l'une des revendications 1 à 8 pour la décoloration de substrats colorés avec des colorants d'oxydation et/ou des colorants directs, les substrats contenant des fibres naturelles choisies parmi les fibres kératiniques.

10. Procédé de décoloration réductrice de substrats colorés avec des colorants naturels et/ou synthétiques, les substrats contenant des fibres naturelles choisies parmi les fibres kératiniques, **caractérisé en ce qu'**on applique sur le substrat coloré un agent selon l'une des revendications 1 à 8 et rincé à nouveau après une période d'exposition.
